(19) Europäisches Patentamt

European Patent Office

Office européen des brevets

(11) **EP 1 913 865 A1**

(12) **EUROPEAN PATENT APPLICATION**

(43) Date of publication:
   **23.04.2008 Bulletin 2008/17**

(21) Application number: **07020328.6**

(22) Date of filing: **17.10.2007**

(51) Int Cl.:
   *A61B 1/273* (2006.01)   *A61B 5/06* (2006.01)
   *A61M 25/01* (2006.01)

(84) Designated Contracting States:
   **AT BE BG CH CY CZ DE DK EE ES FI FR GB GR HU IE IS IT LI LT LU LV MC MT NL PL PT RO SE SI SK TR**
   Designated Extension States:
   **AL BA HR MK RS**

(30) Priority: **17.10.2006 JP 2006282066**
   **17.10.2006 JP 2006282067**

(71) Applicants:
   • **Uchihashi Estec Co., Ltd.**
   **Chuo-ku**
   **Osaka-shi**
   **Osaka 542-0082 (JP)**
   • **Nipro Corporation**
   **Osaka-shi, Osaka 531-8510 (JP)**

(72) Inventors:
   • **Toyoda, Kazumi**
   **Osaka-shi**
   **Osaka 542-0082 (JP)**
   • **Muranaga, Yousuke**
   **Osaka-shi**
   **Osaka 542-0082 (JP)**
   • **Hiejima, Katsuhiro**
   **Osaka-shi**
   **Osaka 531-8510 (JP)**

(74) Representative: **Hofer, Dorothea et al**
   **Prüfer & Partner GbR**
   **Patentanwälte**
   **Sohnckestrasse 12**
   **81479 München (DE)**

(54) **Medical tube inserted in body cavity of patient and medical device set using the same**

(57)   The medical tube inserted into a body cavity of a patient includes a flexible tube (1) and a magnet (3) provided at the tip end portion of the tube (1) for electromagnetically detecting the position of the tip end portion of the tube (1) inserted in the body cavity, from outside the body. This magnet (3) includes a plurality of magnet pieces (4) arranged in the length direction of the tube (1) to have a column-like shape as a whole. The direction of magnetic pole of the magnet (3) is set to the length direction of the tube (1). Therefore, since the magnet (3) is divided into a plurality of magnet pieces (4), even a magnet (3) increased in size can be inserted smoothly into a body cavity of a patient.

FIG.1B

**Description**

This nonprovisional application is based on Japanese Patent Applications Nos. 2006-282066 and 2006-282067 filed with the Japan Patent Office on October 17, 2006, the entire contents of which are hereby incorporated by reference.

BACKGROUND OF THE INVENTION

Field of the Invention

The present invention relates to a medical tube and a medical device set using the same, and more particularly to a medical tube inserted in a body cavity of a patient for use, for example, to feed nutrition solution and a medical device set using the same.

Description of the Background Art

In clinical care, a medical tube is sometimes inserted in a body cavity of a patient for medical treatment. In this case, it is essential to confirm whether a tube tip end is positioned at a prescribed point.
For example, in a where a tube is inserted in the stomach through the patient's mouth or nose and treatment is given with nutrient fed through this tube, if the tip end portion of the tube curls up in the esophagus and does not reach the interior of the stomach, the fed nutrient may be sucked out into the patient's lung, leading a fatal accident. Therefore, it is essential to confirm that the tube tip end reaches a prescribed position of the stomach.
Conventionally, the tip end position of a medical tube is checked by fluoroscopy. However, unfortunately, this method requires the patient to move to an X-ray facility and imposes a heavy burden on the patient.
Proposed then is a method of electromagnetically detecting, from outside of the body, a position of a magnet attached to the interior of a tip end of a medical tube inserted in a body cavity of a patient (see, for example, International Publication WO 1995/008130, International Publication WO 1997/048438, Japanese Patent Laying-Open No. 2004-215992).
In this electromagnetic detection method, magnetic field strength H at a position p at an angle $\varphi$, at a distance R from a magnet having magnetic moment M is represented by the following equation (1), and it is utilized that magnetic field strength H changes according to position p.

$$H = M \, (1+3\cos^2\varphi)^{1/2} / (4\pi\mu_0 R^3) \qquad \ldots \, (1)$$

However, in the medical field, there exist geomagnetism as well as external magnetic fields based on remanence of iron-based structures or electromagnetic waves produced from peripheral equipment, and they act as noises in detection of magnetic field strength H. Therefore, it is requested that S/N ratio should be increased by increasing magnetic moment M of the magnet.
Furthermore, a magneto-impedance effect sensor has recently been developed which has such high sensitivity in that magnetic field detection resolution is $10^{-5}$Oe even with an element length of 2 mm or shorter.
The inner diameter of a medical tube is usually 3 mm, and the outer diameter of the magnet inserted and attached in the tip end portion of the medical tube is about 3 mm. Magnetic moment M of the magnet is represented by M = ml where the strength of magnetic pole is m and the length of the magnet is 1. The strength m of the magnetic pole is dependent on cross section S of the magnet and residual flux Br at the time of magnetization, where residual flux Br is determined by a magnet material.
According to the result of elaborate experiments by the present inventors, in order to effectively detect the position of a magnet inserted and attached in a tip end portion of a medical tube (inner diameter of 3 mm) by a magneto-impedance effect sensor, in a case of a cylindrical magnet having surface residual flux density of 330 mT and made of NiFeB, it is necessary to set the outer diameter to 3 mm$\varphi$ and set the length to 30 mm.
However, insertion of a magnet of such a size into a body cavity through nose, mouth or throat is difficult and imposes a heavy burden on the patient.

SUMMARY OF THE INVENTION

A main object of the present invention is therefore to provide a medical tube which is able to accurately detect a magnet position and can be inserted smoothly into a body cavity of a patient, and medical device set using the same.
In accordance with the present invention, a medical tube inserted into a body cavity of a patient includes: a flexible tube; and a magnet provided at a tip end portion of the tube for electromagnetically detecting a position of the tip end portion of the tube inserted into the body cavity, from outside the body. The magnet includes a plurality of magnet pieces arranged in a length direction of the tube to have a column-like shape as a whole. The direction of magnetic pole of the magnet is set to the length direction of the tube.

Thus, since the magnet is divided into a plurality of magnet pieces, even a magnet increased in size to obtain large magnetic moment can be inserted smoothly into a body cavity of a patient.

Preferably, each of the plurality of magnet pieces is formed like a column.

Preferably, the plurality of magnet pieces are arranged not in contact with each other.

Preferably, the magnet further includes a cushion member provided between each of the plurality of magnet pieces for adjusting flexural rigidity of the tip end portion of the tube.

Preferably, the cushion member has a magnetic property.

Preferably, the plurality of magnet pieces are inserted into the tip end portion of the tube, and each magnet piece is fixed at a prescribed position inside the tube.

Preferably, the entire length of the magnet is 20-50 mm, and the outer diameter of the magnet is 1-5 mm.

A medical device set in accordance with the present invention includes: the medical tube as described above; and a position detector detecting a position of the magnet.

Preferably, the position detector includes a substrate, a pair of magneto-impedance effect elements mounted in parallel and separated by a prescribed distance on the substrate, and a detection circuit detecting a difference of impedance between the pair of magneto-impedance effect elements.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1A and Fig. 1B are views showing a configuration of a medical tube in accordance with an embodiment of the present invention.

Fig. 2 is a view showing an effect of the medical tube shown in Figs. 1A and 1B.

Fig. 3 is a cross-sectional view showing a tip end portion of the medical tube shown in Figs. 1A and 1B.

Fig. 4 is a circuit block diagram showing a configuration of a position detector detecting a position of a magnet shown in Figs. 1A and 1B.

Figs. 5A-5C are graphs showing the characteristics of a magneto-impedance effect element shown in Fig. 4.

Fig. 6 is a view showing that a pair of magneto-impedance effect elements shown in Fig. 4 are mounted on a substrate.

Figs. 7A-7C are views showing that the magneto-impedance effect element shown in Fig. 4, a negative feedback winding and a bias magnetic field winding are mounted on a substrate.

Fig. 8 shows the relation between magnetic moment of a magnet and a magnetic field acting on the magneto-impedance effect element.

Fig. 9 is a graph showing an operation of the position detector shown in Fig. 4.

Figs. 10A and 10B are views showing how to use a medical device set shown in Fig. 1A-Fig. 9.

Figs. 11A and 11B are views showing a modification of the embodiment.

DESCRIPTION OF THE PREFERRED EMBODIMENTS

In a medical tube in accordance with the present invention, a magnet is formed by arranging a plurality of magnet pieces in the length direction of the tube to have a column-like shape as a whole, and the direction of magnetic pole of the magnet is oriented in the length direction of the tube. Therefore, magnetic moment M of the entire magnet is proportional to n1, where the length of a magnetic piece is 1 and the number of magnetic pieces is n. Therefore, a prescribed S/N ratio can be obtained by selecting the number n of magnetic pieces, thereby enhancing the detection accuracy.

Furthermore, when bending moment acts on the tip end portion of the tube, the row of magnet pieces is bent by flexural rigidity of the tube itself. Since the flexural rigidity of the tube itself is low, the aforementioned bending moment can be kept sufficiently low and sufficient flexibility can be obtained. Therefore, the medical tube can be inserted smoothly through nose, mouth and throat.

In addition, provision of a cushion member between the magnet pieces allows the flexural rigidity of the tube tip end portion to be adjusted. Furthermore, imparting a magnetic property to the cushion member prevents leakage of magnetic flux from between the magnet pieces and prevents detection errors resulting from the leakage flux.

Here, the entire length nl of the magnet is set longer than the inner diameter of an organ into which the tube is inserted, so that the orientation of the magnet always agrees with the longitudinal direction of the organ. Therefore, only two-dimensional detection is required, resulting in simpler detection operation and structure. In the following, an embodiment of the present invention will be described in detail with reference to the figures.

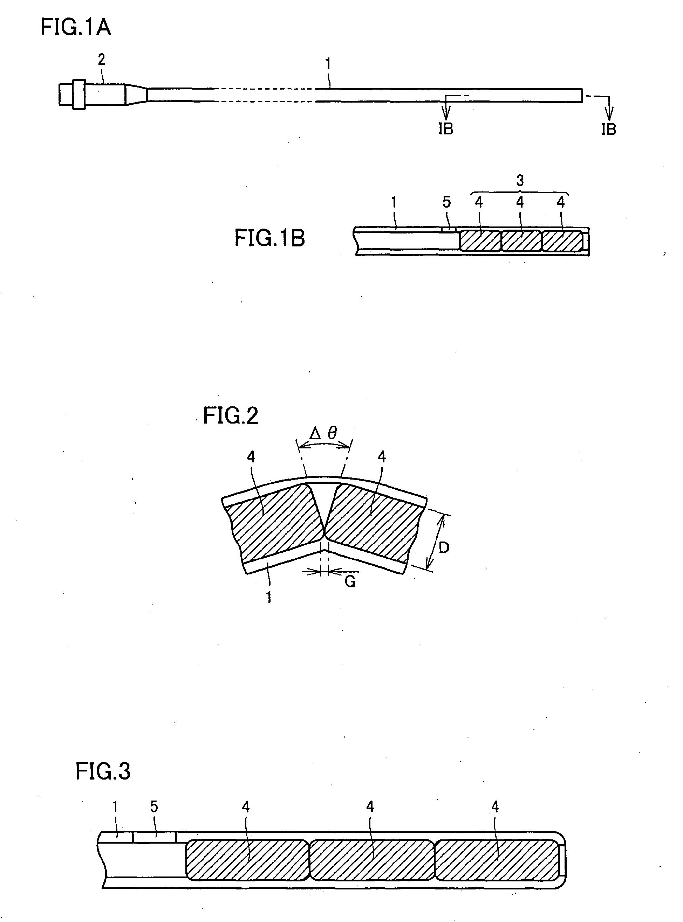

Fig. 1A is a side view showing a configuration of a medical tube in accordance with an embodiment of the present invention, and Fig. 1B is a cross-sectional view taken along IB-IB in Fig. 1 A.

In Fig. 1A, this medical tube includes a flexible tube 1. As flexible tube 1, a see-through plastic tube, for example, a polyamide tube, a silicone resin tube, a polyethylene tube or the like may be used. The inner diameter of tube 1 is usually 2-5 mm.

A connection portion 2 with another member is provided at a base end portion of tube 1. A magnet 3 is inserted and attached in a tip end portion of tube 1. Magnet 3 includes a plurality (three, in the figure) of magnet pieces 4. Each magnet piece 4 is formed like a cylinder, and the edge of the end face of each magnet piece 4 is processed to have prescribed roundness. A plurality of magnet pieces 4 are arranged in the length direction of tube 1 such that magnet 3 has a cylinder-like shape as a whole. A side hole 5 is opened at the base end side of the magnet insertion portion of tube 1. This side hole 5 is used, for example, as a discharge opening of nutrition solution flowing in tube 1.

This medical tube can be used as a feeding tube as well as a urinary catheter, an expansion catheter, a nasogastric tube, an endotracheal tube, a gastric pump tube, a rectal tube, a tube for urinary organs, and the like. In medical treatment, this medical tube is inserted into an organ such as a digestive organ from an insertion starting part such as the patient's nose, mouth or throat. During insertion or after insertion, whether the magnet is positioned at a prescribed point of the organ is detected by a position detector as described later.

When the tip end portion of the medical tube passes through a bending part such as a nose, mouth or throat during insertion, bending moment acts on the tip end portion. In this case, as shown in Fig. 2, a one-side opened gap is formed between the magnet pieces 4 and 4, and gap G based on the roundness of the edge of magnet piece 4 is enlarged to gap $D \times \Delta\theta$ (where D is the diameter of magnet piece 4) based on angle $\Delta\theta$ between the magnet pieces 4 and 4. Accordingly, flexible tube 1 is locally stretched. However, since Young's modulus of flexible tube 1 is small, the tensile stress against the stretch is small and the bending moment can be kept low enough. Therefore, the medical tube can smoothly pass through even such a bending part as nose, mouth or throat, so that pain given to the patient can be alleviated enough.

If slip occurs at a contact interface between magnet piece 4 and tube 1 by local tensile stress when bending moment acts on the tip end portion of flexible tube 1, the original state does not recover from the slippage even after the bending moment is released, and the bend remains at the tip end portion of tube 1. Therefore, the contact interface between magnet piece 4 and tube 1 is preferably fixed and may be fixed by adhesive. As shown in Fig. 3, the gap between the edges of magnet pieces 4, 4 may be filled with the resin of tube 1. Alternatively, for example, a circumferential groove may be provided on the outer circumference of magnet piece 4 and this groove may be filled with the resin of tube 1.

In this manner, after the tip end portion of the medical tube passes through a bending part such as nose, mouth or throat, the tip end portion returns to the linear state, and thereafter the medical tube can smoothly head for a prescribed part.

It is noted that magnet piece 4 may be formed like a column, such as a prism having a triangular, square or hexagonal cross section, in addition to a cylinder. Furthermore, magnet piece 4 may not always be formed like a column and may be granular. Magnet 3 may be shaped like a column as a whole with a plurality of granular magnet pieces 4. The material used for magnet piece 4 is based on Fe with addition ofNi, Co, Cu, A1, B, or the like.

The strength of magnet pole of magnet piece 4 increases in proportion to residual flux density $B_r$ under a saturation magnetic field. More specifically, magnetic moment M is represented by $M = Sn1B_r$, where the cross section of magnet piece 4 is S, the number of magnet pieces 4 is n, and the length of magnet piece 4 is 1. Magnetic moment M can be increased by increasing the number n of magnet pieces 4, and the position of magnet 3 can be detected with a sufficiently high S/N ratio.

As for the size of magnet 3, it is preferable that the entire length is 20-50 mm and the outer diameter is 1-5 mm. Magnet 3 having such an entire length is larger than the inner diameter of the patient's organ, so that the direction of magnet pole of magnet 3 always agrees with the longitudinal direction of the organ. Therefore, detection of magnet 3 can be performed two-dimensionally, thereby simplifying the detection operation of magnet 3 and the structure of the detector.

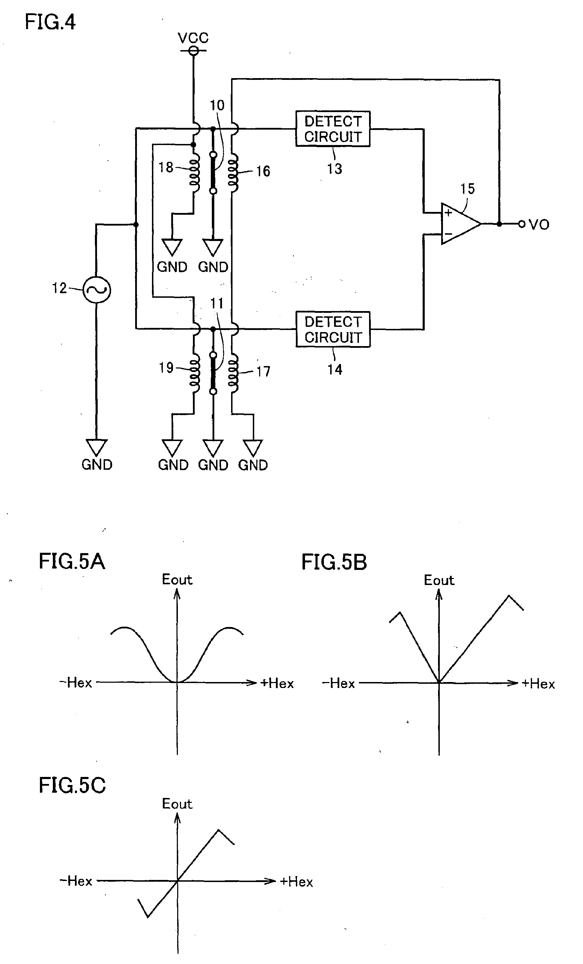

Fig. 4 is a circuit diagram showing a configuration of a position detector detecting the position of magnet 3. The medical tube shown in Fig. 1A-Fig. 3 and the position detector in Fig. 4 constitute a medical device set. In Fig. 4, this position detector includes a pair of magneto-impedance effect elements 10, 11. Each of magneto-impedance effect elements 10, 11 includes an amorphous magnetic wire with zero-magnetostriction or negative-magnetostriction. First and second domains exist in an outer shell portion of this wire, which are alternately provided in the longitudinal direction of the wire and separated by a domain wall. The directions of spontaneous magnetization of the first and second domains are the circumferential direction of the wire and are opposite to each other.

The inductance voltage component of voltage produced between the opposite ends of the wire when high-frequency magnetizing current is fed in such an amorphous magnetic wire results from that the aforementioned easily-magnetizable outer shell portion is magnetized in the circumferential direction by a circumferential magnetic flux produced in the cross section of the wire. Therefore, the magnetic permeability $\mu_\theta$ in the circumferential direction of the wire depends on magnetization in the circumferential direction of the outer shell portion of the wire.

When a signal magnetic field is exerted in the axial direction of the amorphous magnetic wire during conduction, the direction of the magnetic flux acting on the outer shell portion having an easy magnetization characteristic in the circumferential direction is shifted from the circumferential direction by a combination of the circumferential magnetic flux resulting from conduction and the signal magnetic field flux, and the magnetization in the circumferential direction is less likely to occur, accordingly.

Therefore, magnetic permeability $\mu_\theta$ in the circumferential direction of the wire changes and the inductance voltage component varies. This variation phenomenon is referred to as a magneto-inductance effect, and it can be said that this is a phenomenon in which high-frequency magnetizing current (carrier wave) is modulated by a signal magnetic field (signal wave).

Furthermore, when the frequency of conducting current is on the order of MHz, the influence of high frequency skin effect is increased and a skin depth $\delta = 2(\rho/w\mu_\theta)^{1/2}$ ($\mu_\theta$ represents the circumferential magnetic permeability, $\rho$ represents electrical resistance ratio, and w represents angular frequency) changes according to $\mu_\theta$. This $\mu_\theta$ changes according to the signal magnetic field, as described above, and therefore the resistance voltage component in the voltage between opposite ends of the wire also changes according to the signal magnetic field. This variation phenomenon is referred to as a magneto-impedance effect, and it can be said that this is a phenomenon in which high-frequency magnetizing current (carrier wave) is modulated by a signal magnetic field (signal wave).

This position detector also includes a high frequency current source circuit 12 feeding high-frequency magnetizing current to magneto-impedance effect elements 10, 11, detect circuits 13, 14 demodulating a modulated wave produced by modulating high-frequency magnetizing current (carrier wave) by a signal magnetic field (signal wave) acting in the axial direction of magneto-impedance effect elements 10, 11, and an operational differential amplifier 15 differentially amplifying an output voltage of detect circuits 13, 14. The positional relation between magnet 3 and magneto-impedance effect elements 10, 11 can be known from output voltage VO of operational differential amplifier 15. This voltage VO is negatively fed back to magneto-impedance effect elements 10, 11 through negative-feedback windings 16, 17. In addition, a bias magnetic field is applied from bias magnetic field windings 18, 19 to magneto-impedance effect elements 10, 11.

In magneto-impedance effect elements 10, 11, the direction of magnetic flux acting on the outer shell portion having an easy magnetization characteristic in the circumferential direction is shifted from the circumferential direction by a combination of the circumferential magnetic flux based on the magnetizing current and the axial magnetic flux based on the signal magnetic field, so that circumferential magnetic permeability $\mu_\theta$ changes, the inductance is varied, and the impedance is varied by a change in skin depth of high-frequency skin effect of this circumferential magnetic permeability $\mu_\theta$. Therefore, when the direction of the signal magnetic field changes positively or negatively, the circumferential shift $\varphi$ due to the combined magnetic field also changes positively or negatively, but the reduction rate $\cos(\pm\varphi)$ of the magnetic field in the circumferential direction does not change and the reduction degree of $\mu_\theta$ is not changed by either direction of the signal magnetic field. Therefore, the signal magnetic field - output characteristic is approximately symmetric with respect to the y-axis, as shown in Fig. 5A, where the signal magnetic field Hex is plotted along the x-axis and the output voltage Eout of the magneto-impedance effect element is plotted along the y-axis.

The signal magnetic field - output characteristic is non-linear. The non-linear characteristic leads to instability and makes high-sensitivity measurement difficult. Therefore, negative feedback is applied by negative-feedback windings 16, 17 in order to make the output characteristic linear, as shown in Fig. 5B. However, the polarity determination of the signal magnetic field cannot be made with this output characteristic, and therefore, a bias magnetic field is applied by bias windings 18, 19 to enable the polarity determination, as shown in Fig. 5C. In other words, the characteristic in Fig. 5B is moved to the negative direction of the x-axis by a bias magnetic field (-Hb) as shown in Fig. 5C so that the maximum detection range of the signal magnetic field falls within the range of a simple oblique line, -Hmax to +Hmax.

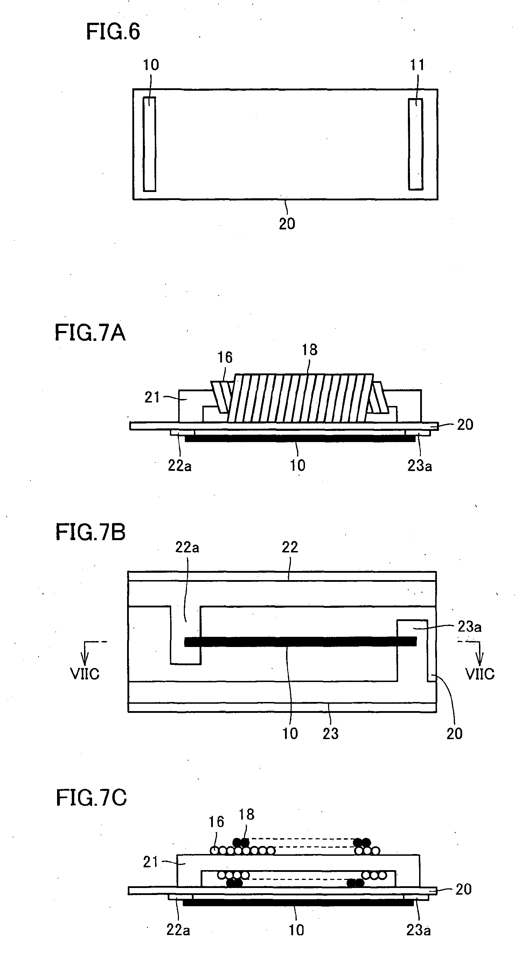

A pair of magneto-impedance effect elements 10, 11 are respectively mounted on one end portion and the other end portion of a strip-like substrate 20, as shown in Fig. 6. The orientation of magneto-impedance effect elements 10, 11 is set to the direction (the width direction of substrate 20) at the right angle with respect to the direction of the line between the center points of elements 10, 11. As long as the angles of orientation of magneto-impedance effect elements 10, 11 are the same, the orientation of magneto-impedance effect elements 10, 11 may be a direction at an angle different from the right angle with respect to the direction of the line between the center points of elements 10, 11.

Here, as magneto-impedance effect elements 10, 11, an alloy composed of a transition metal and 10-30 atomic % of a nonmetal may be used. In particular, a composition including Fe and Co as transition metals and B and Si as nonmetals or a composition including Fe as a transition metal and B and Si as nonmetals may be used. For example, a composition of $Co_{70.5}B_{15}Si_{10}Fe_{4.5}$ may be used. Furthermore, the one having a length of 2000 $\mu$m-6000 $\mu$m and an

outer diameter of 30 $\mu$m-50 $\mu$m$\varphi$ may be used. As magneto-impedance effect elements 10, 11, not only an amorphous magnetic wire having zero-magnetostriction or negative-magnetostriction but also an amorphous ribbon, an amorphous sputter film, or the like may be used.

As high-frequency magnetizing current fed to magneto-impedance effect elements 10, 11, for example, usual high-frequency current such as continuous sinusoidal wave, pulse wave, and triangular wave may be used. As high-frequency magnetizing current source 12, for example, not only a usual oscillator circuit such as a Hartley oscillator circuit, a Colpitts oscillator circuit, a tuned-collector oscillator circuit, and a tuned-base oscillator circuit but also a triangular wave generator integrating a rectangular wave output of a quartz oscillator by an integrating circuit through a direct-current blocking capacitor and amplifying a triangular wave of this integration output by an amplification circuit, or a triangular wave generator using CMOS-IC as an oscillation portion may be used.

Furthermore, as detect circuits 13, 14, for example, a circuit half-wave rectifying a modulated wave by an operational amplification circuit and processing this half-wave rectified wave by a parallel RC circuit or an RC low-pass filter for obtaining an envelop output of the half-wave rectified wave, or a circuit half-wave rectifying a modulated wave by a diode and processing this half-wave rectified wave by a parallel RC circuit or an RC low-pass filter for obtaining an envelop output of the half-wave rectified wave may be used.

In addition, as a detect method, tuning detection may be used, in which a signal wave is sampled by multiplying a modulated wave by a square wave with frequency fs tuned to a modulated wave (frequency fs).

In the example in Fig. 4, a magnetic field to be detected is taken out by demodulation of a modulated wave. However, the present invention is not limited thereto, and any appropriate detect means may be used as long as it can detect a signal magnetic field from a high-frequency magnetizing current wave (carrier wave) modulated by a signal magnetic field (signal wave) acting on magneto-impedance effect elements 10 ,11.

Negative feedback winding 16 (or 17) and bias magnetic field winding 18 (or 19) can be wound around magneto-impedance effect element 10 (or 11). As shown in Figs. 7A-7C, negative feedback winding 16 and bias magnetic field winding 18 can be wound around an iron core 21 forming a loop magnetic circuit with magneto-impedance effect element 10.

Fig. 7A is a side view showing magneto-impedance effect element 10 and the vicinity thereof, Fig. 7B is a bottom view thereof, and Fig. 7C is a cross-sectional view taken along VIIC-VIIC in Fig. 7B.

In Figs. 7A-7C, substrate 20 is formed, for example, of a ceramic plate. Two electrodes 22, 23 are provided on the back surface of substrate 20, and protrusion portions 22a, 23 a for connecting magneto-impedance effect element 10 are respectively provided for electrodes 22, 23. Electrodes 22, 23 are provided by printing or baking of conductive paste, for example, sliver paste.

One end portion and the other end portion of magneto-impedance effect element 10 are respectively connected to protrusion portions 22a, 23 a by soldering or welding. Iron core 21 is provided on substrate 20 at the back side of magneto-impedance effect element 10. Iron core 21 is a C-type iron core made of iron or ferrite. Iron core 21 having approximately the same length as magneto-impedance effect element 10 is provided to be oriented in the same direction as element 10. Any material may be used for iron core 21 as long as it is a magnetic material having small residual flux density. For example, permalloy, ferrite, iron, and amorphous magnetic alloy as well as magnetic material powder blended plastic and the like may be used.

Negative feedback winding 16 is wound around iron core 21 and bias magnetic field winding 18 is wound thereon. The leg portions at opposite ends of iron core 21 are fixed on the surface of substrate 20 by adhesive or the like so that magneto-impedance effect element 10 and iron core 21 constitute a loop magnetic circuit. Here, although magneto-impedance effect element 10 and windings 16, 18 have been described in Figs. 7A-7C, magneto-impedance effect element 11 and windings 17, 19 are configured in a similar manner.

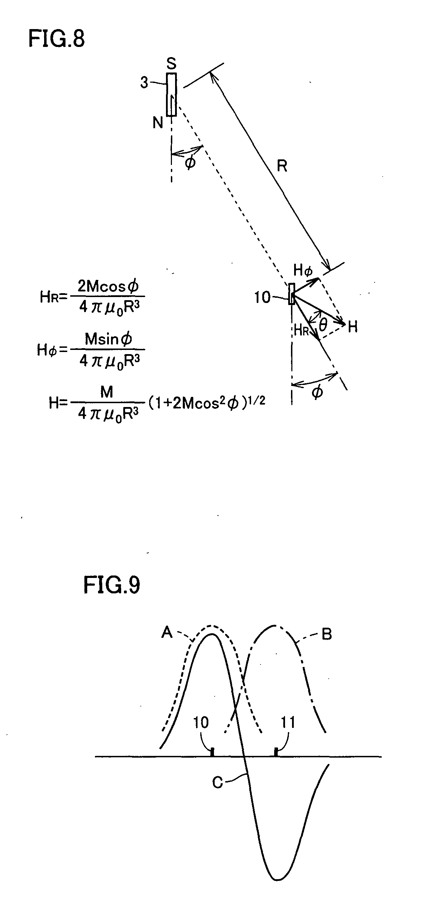

Now, an operation of this position detector will be described. As shown in Fig. 8, given that the magnetic moment of magnet 3 is M and the center of magneto-impedance effect element 10 is present at a position o at distance R and angle $\varphi$ with respect to magnetic moment M, magnetic field strength H at position o based on magnetic moment M is given by the above-noted equation (1).

As is clear from Fig. 8, axial component $H_m$ of magneto-impedance effect element 10 of this magnetic field H is given by the following equation (2).

$$H_m = H\cos(\varphi+\theta) = H(\cos\varphi\cos\theta - \sin\varphi\sin\theta) \dots (2)$$

Here, based on the relation of the following equations (3)(4), Hm can be represented by a formula (5).

$$\sin\theta = \sin\varphi/(1+3\cos^2\varphi)^{1/2} \qquad \ldots (3)$$

$$\cos\theta = 2\cos\varphi/(1+3\cos^2\varphi)^{1/2} \quad \ldots (4)$$

$$H_m = M(\cos^2\varphi+1)/(4\pi\mu_oR^3) \quad \ldots (5)$$

In Fig. 9, assuming that the position of magneto-impedance effect element 10 is at x = 0, when magneto-impedance effect element 10 from side to side with respect to that point, the sensed magnetic filed Hma based on formula (5) of magneto-impedance effect element 10 changes according to curve A. Curve A is acute-angled because of the multiplication effect of $(\cos^2\varphi+1)$. On the other hand, assuming that the position of magneto-impedance effect element 11 is present on the x-axis at a prescribed distance from magneto-impedance effect element 10, the sensed magnetic filed Hmb based on formula (5) of magneto-impedance effect element 11 changes according to curve B having the same shape as curve A. Therefore, a difference Hmab between the sensed magnetic fields of magneto-impedance effect elements 10, 11 changes according to curve C, and when magnet 3 is positioned in the middle between magneto-impedance effect elements 10 and 11, Hmab becomes 0.

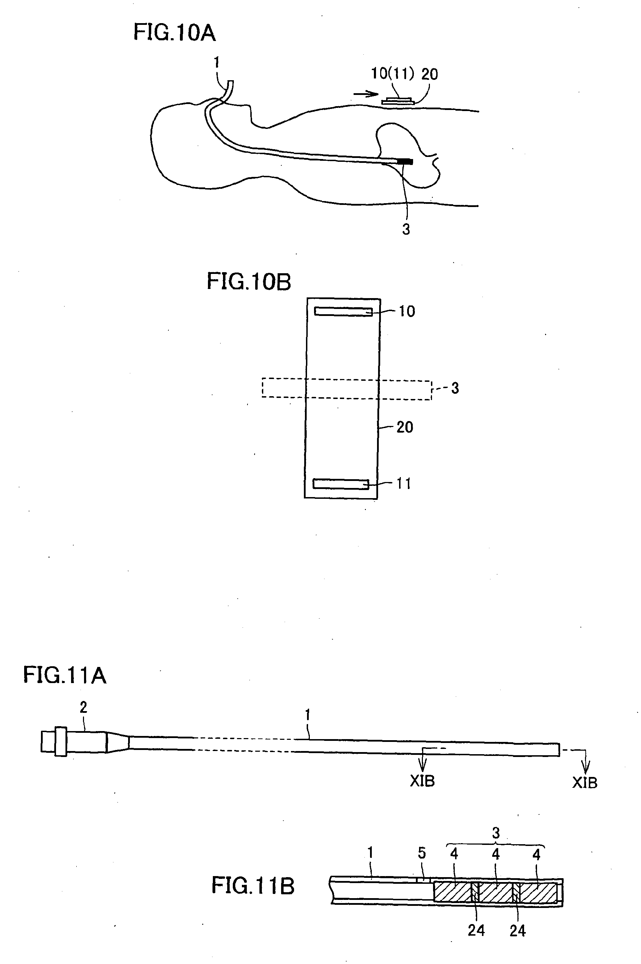

Figs. 10A and 10B are views showing how to use this medical device set. In Figs. 10A and 10B, tube 1 is inserted from the mouth or nose into the stomach of a patient lying face up. Magnet 3 is attached to the tip end of tube 1. Substrate 20 having magneto-impedance effect elements 10, 11 mounted thereon is arranged in parallel with the surface of the patient's belly, and the longitudinal direction of substrate 20 is oriented at right angles to the moving direction of substrate 20.

If the entire length of magnet 3 is equal to or greater than the diameter of an organ, the direction of magnet 3 is restricted to the longitudinal direction of the organ, so that the directional range of the tip end portion of tube 1 is determined according to the applications described above. Therefore, the magnetization direction of magnet 3 can be specified based on the orientation. If the magnetic sensing direction of magneto-impedance effect elements 10, 11 of the position detector is arranged in parallel with the orientation, output voltage VO of operational differential amplifier 15 becomes 0 when magnet 3 is positioned in the middle between magneto-impedance effect elements 10 and 11.

Therefore, the position of magnet 3 can be detected by moving substrate 20 in the horizontal direction on the patient's belly and finding the position where output voltage VO of operational differential amplifier 15 becomes 0. Conversely, with substrate 20 arranged on the patient's stomach, tube 1 may be inserted until output voltage VO of operational differential amplifier 15 becomes 0.

Here, when the distance between magneto-impedance effect elements 10 and 11 is set to 10-30 cm, the change in the vicinity of the 0 point of output voltage VO of operational differential amplifier 15 is steep, so that the 0 point, that is, the position of magnet 3 can be detected accurately.

Furthermore, when the entire position detector shown in Fig. 4 is mounted on substrate 20, the apparatus can be miniaturized. Alternatively, only magneto-impedance effect elements 10, 11 and windings 16-18 of the position detector shown in Fig. 4 may be mounted on substrate 20 and the other parts may be mounted on another substrate, and then the two substrates may be connected to each other by a flexible lead. In this case, the portion moved on the patient's belly can be reduced in weight.

Figs. 11A and 11B are views showing a modification of the present embodiment, in contrast with Figs. 1A and 1B. Fig. 11B is a cross-sectional view taken along XIB-XIB in Fig. 11A. Referring to Figs. 11A and 11B, this medical tube differs from that of Figs. 1 A and 1B in that a cushion member 24 is provided between magnet pieces 4 and 4. A spring, rubber, foam (foamed plastic, foamed rubber) or the like may be used as cushion member 24. Thus, the flexural rigidity of the tip end portion of tube 1 can be adjusted.

Furthermore, a magnetic property may be imparted to cushion member 24. In other words, a metal spring having a magnetic property may be used as cushion member 24, or rubber, foamed plastic, foamed rubber or the like with addition of magnetic powders may be used. In this case, flux leakage from between magnet pieces 4 and 4 can be prevented, thereby preventing a detection error caused by magneto-impedance effect elements 10, 11 sensing that leakage flux.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A medical tube inserted into a body cavity of a patient comprising:

   a flexible tube (1); and
   a magnet (3) provided at a tip end portion of said tube (1) for electromagnetically detecting a position of the tip end portion of the tube (1) inserted into said body cavity, from outside the body, wherein

   said magnet (3) includes a plurality of magnet pieces (4) arranged in a length direction of said tube (1) to have a column-like shape as a whole, and
   a direction of magnetic pole of said magnet (3) is oriented in the length direction of said tube (1).

2. The medical tube according to claim 1, wherein each of said plurality of magnet pieces (4) is formed like a column.

3. The medical tube according to claim 1 or 2, wherein said plurality of magnet pieces (4) are arranged not in contact with each other.

4. The medical tube according to one of claims 1 to 3, wherein said magnet (3) further includes a cushion member (24) provided between each of said plurality of magnet pieces (4) for adjusting flexural rigidity of the tip end portion of said tube.

5. The medical tube according to claim 4, wherein said cushion member (24) has a magnetic property.

6. The medical tube according to one of claims 1 to 5, wherein
   said plurality of magnet pieces (4) are inserted into the tip end portion of said tube (1), and
   each magnet piece (4) is fixed at a prescribed position inside said tube (1).

7. The medical tube according to one of claims 1 to 6, wherein
   an entire length of said magnet (3) is 20-50 mm, and
   an outer diameter of said magnet (3) is 1-5 mm.

8. A medical device set comprising:

   the medical tube according to claim 1; and
   a position detector detecting a position of said magnet (3).

9. The medical device set according to claim 8, wherein
   said position detector includes

   a substrate (20),
   a pair of magneto-impedance effect elements (10, 11) mounted in parallel and separated by a prescribed distance on said substrate (20), and
   a detection circuit (12-19) detecting a difference of impedance between said pair of magneto-impedance effect elements (10, 11).

FIG.1A

FIG.1B

FIG.2

FIG.3

**FIG.4**

**FIG.5A**

**FIG.5B**

**FIG.5C**

**FIG.6**

**FIG.7A**

**FIG.7B**

**FIG.7C**

FIG.8

$$H_R = \frac{2M\cos\phi}{4\pi\mu_0 R^3}$$

$$H_\phi = \frac{M\sin\phi}{4\pi\mu_0 R^3}$$

$$H = \frac{M}{4\pi\mu_0 R^3}(1+2M\cos^2\phi)^{1/2}$$

FIG.9

FIG.10A

1

10(11) 20

3

FIG.10B

10

3

20

11

FIG.11A

2

1

XIB

XIB

3

1    5    4    4    4

FIG.11B

24    24

**European Patent Office**

## EUROPEAN SEARCH REPORT

Application Number

EP 07 02 0328

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/072662 A1 (HALL ANDREW F [US] ET AL) 13 June 2002 (2002-06-13) | 1-7 | INV. A61B1/273 A61B5/06 A61M25/01 |
| Y | * paragraphs [0029], [0034], [0036], [0037]; figures 8,10-13 * | 8,9 | |
| X | JP 2000 175865 A (HONDA SEIKI KK; TANAHASHI YOSHIKATSU; ISHIYAMA KAZUYUKI; ARAI KENICHI) 27 June 2000 (2000-06-27) | 1-4 | |
| Y | * figure 1 * | 8,9 | |
| X | US 6 902 528 B1 (GARIBALDI JEFFREY M [US] ET AL) 7 June 2005 (2005-06-07) | 1-7 | |
| Y | * column 5, lines 8-17; figure 7 * | 8,9 | |
| Y | EP 1 438 921 A (UCHIHASHI ESTEC CO LTD [JP]; IKEUCHI KEN [JP]) 21 July 2004 (2004-07-21) * paragraphs [0013], [0022], [0023]; claims 1-4; figure 1 * | 8,9 | |

TECHNICAL FIELDS SEARCHED (IPC)

A61B
A61M

The present search report has been drawn up for all claims

| Place of search | Date of completion of the search | Examiner |
|---|---|---|
| The Hague | 27 February 2008 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 07 02 0328

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2008

| Patent document cited in search report | | Publication date | Patent family member(s) | | Publication date |
|---|---|---|---|---|---|
| US 2002072662 | A1 | 13-06-2002 | NONE | | |
| JP 2000175865 | A | 27-06-2000 | NONE | | |
| US 6902528 | B1 | 07-06-2005 | AU | 4237600 A | 14-11-2000 |
| | | | WO | 0060996 A1 | 19-10-2000 |
| | | | US | 2005033162 A1 | 10-02-2005 |
| EP 1438921 | A | 21-07-2004 | JP | 2004215992 A | 05-08-2004 |
| | | | US | 2004143183 A1 | 22-07-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006282066 A
- JP 2006282067 A
- WO 1995008130 A
- WO 1997048438 A
- JP 2004215992 A